# EUROPÄISCHE PATENTANMELDUNG

(11) **EP 3 944 887 A1**
(43) Veröffentlichungstag der Anmeldung: **02.02.2022**
(21) Anmeldenummer: 20188831.0
(22) Anmeldetag: 31.07.2020
(51) Int. Cl.: B01D 39/10, B01D 39/16, A62B 23/02

(54) **FASERMATERIALVERBUND MIT REAKTIVEN SAUERSTOFFSPEZIES NEUTRALISIERENDEM BEREICH**

(71) Anmelder: Siemens Aktiengesellschaft, 80333 München (DE)
(72) Erfinder: Jensen, Jens Dahl, 14050 Berlin (DE); Krüger, Ursus, 14089 Berlin (DE); Rueda, Ana-Isabel, 80339 München (DE); Winkler, Gabriele, 13587 Berlin (DE)

(57) **Zusammenfassung**

Die Erfindung betrifft einen Fasermaterialverbund (10). Um einen verbesserten Fasermaterialverbund (10) anzugeben weist dieser zumindest einen ersten Bereich (14), der zum Erzeugen von reaktiven Sauerstoffspezies (ROS) ausgebildet ist und einen zweiten Bereich (16), der zum Neutralisieren der reaktiven Sauerstoffspezies (ROS) ausgebildet ist, auf. Weiterhin betrifft die Erfindung einen Mund-Nasen-Schutz (50) sowie eine persönliche Schutzausrüstung.

## Beschreibung

Die Erfindung betrifft einen Fasermaterialverbund, einen Mund-Nasen-Schutz sowie eine persönliche Schutzausrüstung

Ein derartiger Fasermaterialverbund kann beispielsweise in antibakteriellen und/oder antiviralen Filtern oder chirurgischen Mund-Nasen-Schutzmasken zum Einsatz kommen. Weiterhin kann das Fasermaterial in Filtern für Atemschutzvorrichtungen oder in Klimaanlagen, die wechselnden Umgebungstemperaturen ausgesetzt sind, eingesetzt werden.

Aus der unveröffentlichten Anmeldung DE 10 2020 203 783.3 ist ein Fasermaterial für eine antibakterielle und/oder antivirale Verwendung bekannt, das Fasern aufweist, die metallisches Silber und Mangan(IV)-oxid aufweisen. Das Fasermaterial ist dabei z. B. zur Verwendung für einen gasdurchlässigen Filter und/oder Mund-Nasen-Schutz ausgebildet. Es hat sich dabei herausgestellt, dass das Silber und das Mangan (IV)-oxid sehr gut auf Fasern aufgetragen werden können und mit der eigentlich unerwünschten Feuchtigkeit der Atemluft bzw. Umgebungsluft sogenannte reaktive Sauerstoffspezies bilden, die auch als ROS (= reactive oxygen species) bekannt sind.

Es ist Aufgabe einen verbesserten Fasermaterialverbund anzugeben. Weiterhin ist es Aufgabe der Erfindung einen verbesserten Mund-Nasen-Schutz anzugeben, sowie eine verbesserte persönliche Schutzausrüstung.

Diese Aufgabe wird durch einen Fasermaterialverbund mit den im Anspruch 1 angegebenen Merkmalen gelöst. Der Fasermaterialverbund weist dazu zumindest einen ersten Bereich auf, der zum Erzeugen von reaktiven Sauerstoffspezies ausgebildet ist und einen zweiten Bereich, der zum Neutralisieren der reaktiven Sauerstoffspezies ausgebildet ist. Der reaktive Sauerstoffspezies bildende erste Bereich kann dabei gemäß der eingangs genannten Materialkombination aus metallischem Silber und Mangan(IV)-oxid ausgebildet sein. Die Sauerstoffspezies weisen dabei antivirale und antibakterielle Eigenschaften auf und sollten daher nicht in größeren Mengen mit einem Träger einer Maske in Kontakt kommen oder von diesem aufgenommen werden. Dazu wird ein zweiter Bereich vorgeschlagen, der die verbleibenden reaktiven Sauerstoffspezies neutralisiert. Dies erhöht die Verträglichkeit des Fasermaterials. Das Neutralisieren der reaktiven Sauerstoffspezies kann dabei beispielsweise durch einen Elektronenaustausch zwischen dem zweiten Bereich und den reaktiven Sauerstoffspezies realisiert werden. Dies kann insbesondere mit einem elektrisch leitenden Material oder einem Material, das einen Elektronenaustausch zwischen den reaktiven Sauerstoffspezies und dem zweiten Bereich ermöglicht, realisiert werden.

In einer weiteren Ausführungsform ist der erste Bereich und/oder der zweite Bereich jeweils als eine separate Lage ausgebildet. Dies kann zu einer einfacheren Fertigung der Bereiche führen, indem z. B. als Bahnmaterial ein reaktive Sauerstoffspezies produzierendes Fasermaterial und separat davon ein reaktive Sauerstoffspezies neutralisierendes Material zur Verfügung gestellt wird. Die beiden Materialien können dann zu einem Verbund gefügt, z. B. verklebt, verschweißt, vernäht werden.

In einer weiteren Ausführungsform weist der Fasermaterialverbund einen Zwischenbereich auf, der insbesondere als ein Vlies ausgebildet ist. Der Zwischenbereich ist zwischen dem ersten und dem zweiten Bereich nageordnet und trennt diese voneinander. So kann die antivirale und antibakterielle Wirkung des reaktive Sauerstoffspezies produzierenden Bereichs optimiert werden, während die verbleibenden ROS, die durch den Zwischenbereich dringen, im zweiten Bereich neutralisiert werden können. Der Zwischenbereich kann weiterhin eine Aktivkohleschicht aufweisen, die bereits Teile der reaktiven Sauerstoffspezies neutralisiert.

In einer weiteren Ausführungsform weist der erste Bereich Silber und Mangandioxid auf, insbesondere mit Mangandioxid dotierte Silberpartikel. Es hat sich als besonders effizient erwiesen, einen Fasermaterialverbund mit dieser Materialkombination auszustatten, da diese eine hohe Verfügbarkeit und hohe Verträglichkeit aufweisen.

In einer weiteren Ausführungsform ist der erste Bereich und/oder der zweite Bereich jeweils als ein Vlies, insbesondere ein Polypropylen-Vlies, ausgebildet. Dabei können der erste Bereich und der zweite Bereich auf einem einzigen durchgängigen Vlies auf jeweils gegenüberliegenden Seiten aufgebracht sein.

In einer weiteren Ausführungsform weist der zweite Bereich eine elektrisch leitende Beschichtung auf. So kann ein beschichtetes Fasermaterial für den zweiten Bereich zum Einsatz kommen und eine reaktive Sauerstoffspezies neutralisierende Wirkung der elektrisch leitenden Beschichtung ideal zum Einsatz kommen. Dabei neutralisiert die elektrisch leitende Eigenschaft der Beschichtung die reaktiven Sauerstoffspezies, indem Elektronen mit den reaktiven Sauerstoffspezies ausgetauscht werden.

In einer weiteren Ausführungsform weist der zweite Bereich ein elektrisch leitendes Material auf. So kann ein Geflecht bzw. ein Gewebe aus dem elektrisch leitendem Material zum Einsatz kommen. Alternativ oder ergänzend kann eine dünne, elektrisch leitende Folie, die für Atemluft durchströmbar ist und einen geringen Atemwiderstand aufweist, zum Einsatz kommen. In einer alternativen Ausführungsform kann der zweite Bereich neben weiteren Materialien, z. B. eine hautverträgliche Vliesschicht ein elektrisch leitendes Material aufweisen.

In einer weiteren Ausführungsform weist der zweite Bereich ein Edelstahlgeflecht oder Edelstahlgitter auf. Es hat sich als besonders vorteilhaft erwiesen, ein Edelstahlgeflecht als reaktive Sauerstoffspezies neutralisierenden Bereich einzusetzen. Die Verwendung eines Edelstahls hat den Vorteil, dass diese gut verträglich ist und eine sehr gute neutralisierende Wirkung bzgl. der reaktiven Sauerstoffspezies aufweist. Der Wirkmechanismus basiert hier auch auf einem Elektronenaustausch.

Die Aufgabe wird weiterhin durch einen Mund-Nasen-Schutz gelöst, insbesondere durch eine chirurgische Maske, aufweisend einen erfindungsgemäßen Fasermaterialverbund. Der Mund-Nasen-Schutz weist damit einen Fasermaterialverbund mit zumindest einem ersten Bereich auf, der zum Erzeugen von reaktiven Sauerstoffspezies ausgebildet ist und einen zweiten Bereich, der zum Neutralisieren der reaktiven Sauerstoffspezies ausgebildet ist. Der erste Bereich ist dabei vorteilhaft vom Träger des Mund-Nasen-Schutz abgewandten Seite angeordnet.

In einer weiteren Ausführungsform des Mund-Nasen-Schutz ist der zweite Bereich so angeordnet, dass dieser zwischen einer Mundpartie eines Trägers des Mund-Nasen-Schutz und dem ersten Bereich angeordnet ist. Diese Anordnung stellt sicher, dass die Atemluft des Trägers keine oder nur sehr geringe Mengen an reaktiven Sauerstoffspezies aufweist.

In einer weiteren Ausführungsform ist der erste Bereich als äußerste Schicht auf einer der Mundpartie eines Trägers des Mund-Nasen-Schutz abgewandten Seite angeordnet.

In einer weiteren Ausführungsform weist der Mund-Nasen-Schutz eine hydrophobe Schicht auf, diese kann insbesondere als dem Träger abgewandte Außenschicht des Mund-Nasen-Schutz ausgebildet sein. Auch ist eine solche Schicht denkbar zwischen erstem Bereich und zweiten Bereich, sodass die Feuchtigkeit im ersten Bereich bleibt, was den Effekt der Erzeugung von reaktiven Sauerstoffspezies verstärken kann.

In einer weiteren Ausführungsform weist der Mund-Nasen-Schutz eine hygroskopische Schicht auf. Diese kann in der Nähe des ersten Bereichs angeordnet sein, sodass die Feuchtigkeit den reaktiven Sauerstoffspezies erzeugenden Effekt verstärken kann.

In einer weiteren Ausführungsform ist der Mund-Nasen-Schutz mehrteilig ausgestaltet, sodass der zweite Bereich Bestandteil des Mund-Nasen-Schutz und der erste Bereich austauschbar gestaltet ist. So kann beispielsweise ein gut zu reinigender elektrisch leitender zweiter Bereich 16 aus Edelstahl Teil des Mund-Nasen-Schutz sein, oder sogar als wiederverwendbares Basiselement des Mund-Nasen-Schutz ausgestaltet sein und der erste Bereich auf einem Austauschbaren Filtervlies angeordnet sein, das wiederum gut entsorgbar ist.

Die Aufgabe wird weiterhin durch persönliche Schutzausrüstung aufweisend einen erfindungsgemäßen Fasermaterialverbund gelöst. Dabei ist der zweite Bereich zwischen einer Hautpartie eines Trägers und dem ersten Bereich angeordnet. So können die antivirale und antibakterielle Wirkung auch in einem Kleidungsstück, z. B. ein Labor/Arzt-Kittel zur Anwendung kommen.

Im Folgenden wird die Erfindung anhand der in den Figuren dargestellten Ausführungsbeispiele näher beschrieben und erläutert. Es zeigen:
- FIG 1: einen schematischen Querschnitt eines Fasermaterialverbunds,
- FIG 2: einen schematischen Querschnitt eines weiteren Fasermaterialverbunds,
- FIG 3: eine Ausführungsform eines Mund-Nasen-Schutz und
- FIG 4: eine weitere Ausführungsform eines Mund-Nasen-Schutz.

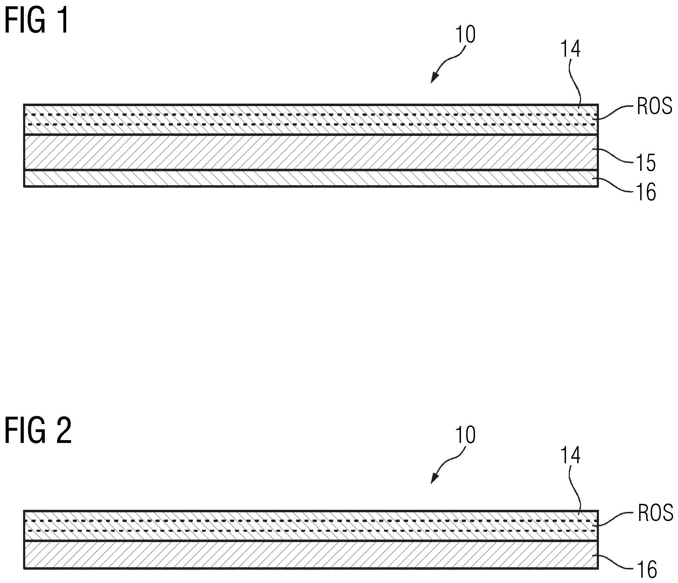

FIG 1 zeigt einen Querschnitt durch einen Fasermaterialverbund 10. Dieser weist einen ersten Bereich 14, einen zweiten Bereich 16 sowie einen zwischen dem ersten und zweiten Bereich 14, 16 liegenden Zwischenbereich 15 auf.

Im ersten Bereich 14 ist schematisch angedeutet, dass im ersten Bereich bereits reaktive Sauerstoffspezies ROS ausgebildet wurden, die sich dort angesammelt haben und die eine antivirale und antibakterielle Wirkung aufweisen.

Es ist denkbar, dass alle Bereiche 14, 15, 16 durch ein einziges Vlies realisiert sind und von den jeweiligen Seiten mit Schichten versehen sind, die die Funktionen des ersten Bereichs 14 und des zweiten Bereichs 16 übernehmen. Der erste Bereich 14 kann also mit einer Silberbeschichtung mit Braunsteindotierung versehen sein und der zweite Bereich 16 mit einer elektrisch leitfähigen Beschichtung. Ebenso denkbar ist, dass Edelstahlfasern/-fäden im zweiten Bereich 16 in das Vlies eingewoben sind.

Weiterhin ist eine Ausgestaltung möglich in der die Bereiche 14, 15, 16 als drei eigene Lagen oder zumindest als zwei Lagen realisiert sind, d.h. eine Lage Vlies für den ersten Bereich 14, die mit einem Materialkomplex versehen ist, der reaktive Sauerstoffspezies ROS produziert, eine Lage Vlies für den Zwischenbereich 15, der als Vlies oder als Vlies mit Aktivkohle ausgestaltet sein kann und eine Lage elektrisch leitendes Gewebe für den zweiten Bereich 16.

FIG 2 zeigt einen Querschnitt durch eine weitere Ausführungsform eines Fasermaterialverbund 1. Hier grenzen der erste Bereich 14 und der zweite Bereich 16 unmittelbar aneinander. Dies kann entweder durch das direkte Verbinden von getrennten Lagen für jeden der Bereiche 14, 16 oder durch auftragen/einbringen der Bereiche 14,16 auf/in ein einziges Vlies erreicht werden. Die so gezeigte Ausführungsform ist dabei kompakter und weist ggf. einen niedrigeren Atemwiderstand auf.

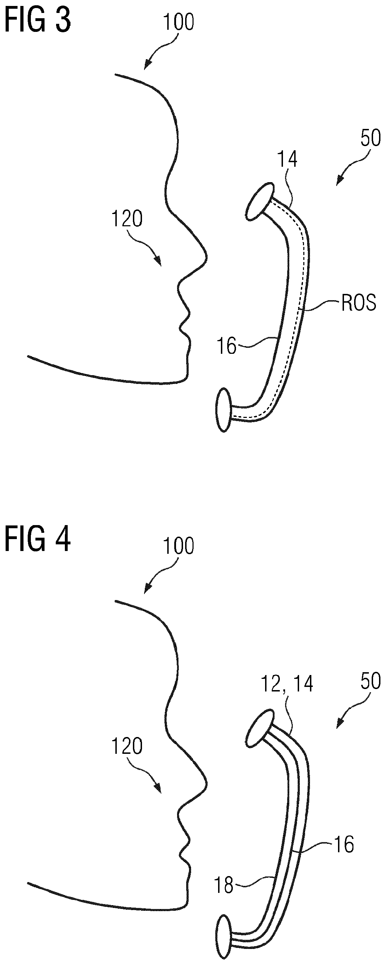

FIG 3 zeigt einen Mund-Nasen-Schutz 50 in einer einfachen Ausführungsform. Der Mund-Nasen-Schutz 50 wird oft auch Mund-Nasen-Schutzmaske bzw. Mundschutzmaske genannt. Der Mund-Nasen-Schutz 50 weist dabei beispielsweise ein oder mehrere Vlies-Schichten auf, die einen ersten Bereich 14 und einen zweiten Bereich 16 aufweisen können. Der erste Bereich 14 ist dabei zum Erzeugen von reaktiven Sauerstoffspezies ROS ausgebildet, z. B. durch eine Beschichtung mit Silberpartikeln, die mit Mangan(IV)oxid dotiert sind. Der zweite Bereich 16 ist dabei zum Neutralisieren der reaktiven Sauerstoffspezies ROS ausgebildet. Einen Träger 100 erreicht so im Bereich einer Mund-Nasen-Partie 120 kurz Mundpartie 120 keine erhöhte Konzentration von reaktiven Sauerstoffspezies ROS. Dies kann die Verträglichkeit des Mund-Nasen-Schutzes 50 bei längeren Tragedauern erheblich verbessern.

FIG 4 zeigt einen weiteren Mund-Nasen-Schutz 50, z. B. auf Basis von Vlies-Schichten. In direktem Kontakt mit der Umgebungsluft ist eine wasserabweisende, hydrophobe Außenschicht 12. Die Außenschicht 12 ist dabei direkt auf den ersten Bereich 14 aufgetragen, sodass nur wenig Flüssigkeit in die Maske 50 dringt. Sollte aber dennoch infektiöses Aerosol oder Tröpfchen in den ersten Bereich 14 gelangen, so wird dieses infektiöse Material durch die im ersten Bereich erzeugten reaktiven Sauerstoffspezies ROS desinfiziert / neutralisiert. Eine dem Träger 100 zugewandte hautverträgliche Schicht 18 kann bspw. als ein hautverträgliches Polypropylen-Vlies ausgebildet sein und liegt unmittelbar auf der Mund-Nasen-Partie 120 des Trägers 100 auf.

Im vorliegenden Ausführungsbeispiel ist der antiviral wirkende erste Bereich 14 als Teil der Außenschicht 12 vorgesehen. Diese bakterizide und viruzide Schicht kann hauptsächlich aus Silber bestehen und ist mit Braunstein (MnO₂) dotiert. Silber und Mangandioxid (Braunstein) bilden aus Sauerstoff und Feuchtigkeit der Atemluft Sauerstoffradikale, die auch als ROS (= reactive oxygen species) bekannt sind.

Diese äußert reaktive Sauerstoffspezis ROS können Proteine, Lipide, RNA oder DNA, aus welchem Bakterien und Viren bestehen, zerstören, indem sie mit diesen eine chemische Reaktion eingehen, sodass Bakterien und Viren unschädlich gemacht werden.

Da überschüssiges ROS auf den Rachenraum des Trägers beim Einatmen schädlich wirken können und die Schleimhäute reizen, wird auf einen zweiten Bereich 16 ein elektrisch leitender Metallfaden aus Edelstahl, der auf die ROS- Radikale reduzierend wirkt, eingearbeitet. Edelstahl wird bevorzugt, weil Edelstahl für den Träger 100 der Mund-Nasen-Schutz 50 unbedenklich ist.

In einer weiteren Ausführungsform könnte die hautverträgliche Schicht 18 zumindest auf der dem Träger 100 abgewandten Seite mit einer antibakteriell und antiviral wirkenden ersten Bereich 14 versehen sein. Dies würde die Wirkung weiter verstärken. Vorzugsweise ist dann in der hautverträglichen Schicht eine weiterer zweiter Bereich 16, z. B. elektrisch Leitend, vorgesehen.

Zusammenfassend betrifft die Erfindung einen Fasermaterialverbund 10. Um einen verbesserten Fasermaterialverbund 10 anzugeben weist dieser zumindest einen ersten Bereich 14, der zum Erzeugen von reaktiven Sauerstoffspezies ROS ausgebildet ist und einen zweiten Bereich 16, der zum Neutralisieren der reaktiven Sauerstoffspezies ROS ausgebildet ist, auf. Weiterhin betrifft die Erfindung einen Mund-Nasen-Schutz 50 sowie eine persönliche Schutzausrüstung.

### Bezugszeichen

- ROS: reaktive Sauerstoffspezies

- 10: Fasermaterialverbund
- 12: Außenschicht
- 14: erster, ROS erzeugender Bereich
- 15: Zwischenbereich
- 16: zweiter, ROS neutralisierender Bereich
- 18: hautverträgliche Schicht
- 50: Mund-Nasen-Schutz

- 100: Träger
- 120: Mund-Nasen-Partie

## Patentansprüche

1. Fasermaterialverbund (10) aufweisend zumindest einen ersten Bereich (14), der zum Erzeugen von reaktiven Sauerstoffspezies (ROS) ausgebildet ist und
einen zweiten Bereich (16), der zum Neutralisieren der reaktiven Sauerstoffspezies (ROS) ausgebildet ist.

2. Fasermaterialverbund (10) nach Anspruch 1, wobei der erste Bereich (14) und/oder der zweite Bereich (16) jeweils als eine separate Lage ausgebildet sind.

3. Fasermaterialverbund (10) nach einem der vorhergehenden Ansprüche, aufweisend einen Zwischenbereich (15), der insbesondere als ein Vlies ausgebildet ist.

4. Fasermaterialverbund (10) nach einem der vorhergehenden Ansprüche, wobei der erste Bereich (14) Silber und Mangandioxid aufweist, insbesondere mit Mangandioxid dotierte Silberpartikel.

5. Fasermaterialverbund (10) nach einem der vorhergehenden Ansprüche, wobei der erste Bereich (14) und/oder der zweite Bereich (16) jeweils als ein Vlies, insbesondere ein Polypropylen-Vlies, ausgebildet sind.

6. Fasermaterialverbund (10) nach einem der vorhergehenden Ansprüche, wobei der zweite Bereich (16) eine elektrisch leitende Beschichtung aufweist.

7. Fasermaterialverbund (10) nach einem der vorhergehenden Ansprüche, wobei der zweite Bereich (16) ein elektrisch leitenden Material aufweist, insbesondere ein elektrisch leitendes Gewebe.

8. Fasermaterialverbund (10) nach einem der vorhergehenden Ansprüche, wobei der zweite Bereich (16) ein Edelstahlgeflecht oder Edelstahlgitter aufweist.

9. Mund-Nasen-Schutz (50), insbesondere chirurgische Maske, aufweisend einen Fasermaterialverbund (10) nach einem der vorhergehenden Ansprüche, wobei der Fasermaterialverbund (10) einen ersten Bereich (14), der zum Erzeugen von reaktiven Sauerstoffspezies (ROS) ausgebildet ist und einen zweiten Bereich (16), der zum Neutralisieren der reaktiven Sauerstoffspezies (ROS) ausgebildet ist, aufweist.

10. Mund-Nasen-Schutz (50) nach Anspruch 9, wobei der zweite Bereich (16) so angeordnet ist, dass dieser zwischen einer Mundpartie (120) eines Trägers (100) des Mund-Nasen-Schutz (50) und dem ersten Bereich (14) angeordnet ist.

11. Mund-Nasen-Schutz (50) nach Anspruch 9 oder 10, wobei der erste Bereich (14) als äußerste Schicht auf einer der Mundpartie (120) eines Trägers (100) des Mund-Nasen-Schutz (50) abgewandten Seite angeordnet ist.

12. Mund-Nasen-Schutz (50) nach einem der Ansprüche 8 bis 11, aufweisend eine hydrophobe Schicht (12).

13. Mund-Nasen-Schutz (50) nach einem der Ansprüche 8 bis 11, aufweisend eine hygroskopische Schicht.

14. Mund-Nasen-Schutz (50) nach einem der Ansprüche 8 bis 12, wobei der zweite Bereich (16) Bestandteil des Mund-Nasen-Schutz (50) ist und der erste Bereich (14) austauschbar gestaltet ist.

15. Persönliche Schutzausrüstung aufweisend einen Fasermaterialverbund (10) nach einem der Ansprüche 1 bis 8, wobei ein zweiter Bereich (16) des Fasermaterialverbunds (10) zwischen einer Hautpartie eines Trägers (100) und einem ersten Bereich (14) angeordnet ist.
